# EUROPEAN PATENT SPECIFICATION

(11) **EP 1 870 113 B1**
(45) Date of publication and mention of the grant of the patent: **03.06.2009**
(21) Application number: 06115778.0
(22) Date of filing: 21.06.2006
(51) Int. Cl.: A61L 2/18, A47L 15/22

(54) **Disinfection device comprising spray means**
Desinfektionsvorrichtung mit Sprühmitteln
Dispositif de désinfection comprenant des moyens d'arrosage

(43) Date of publication of application: 26.12.2007
(73) Proprietor: Getinge Disinfection AB, 351 15 Växjö (SE)
(72) Inventor: Johansson, Jonas, Växjö SE-352 45 (SE)
(74) Representative: Fritsche, Daniel

(56) References cited:
- EP-A1- 0 559 466
- FR-A1- 2 713 078
- JP-A- 2005 328 886
- US-A- 3 413 987
- US-A- 5 488 965
- US-A1- 2004 163 685
- US-B1- 6 571 812

## Description

### Field of the Invention

The present invention relates a disinfection device with a spray arm which is rotatable about a shaft in a disinfection space and which is provided with outlet nozzles for spraying the object that is to be disinfected.

### Background Art

Different types of cleaning machines are available on the market. One category consists of disinfection devices, which exist in different sizes and shapes. They may have the size where it is possible for the user to insert, for example, a trolley containing a rack with one or more baskets holding the objects that are to be disinfected. Alternatively, they can be smaller, in which case only one or more baskets containing the objects that are to be disinfected are inserted into the disinfection chamber. A common feature is, however, that heavy demands are placed on these devices; for instance the device should be able to clean the objects in a satisfactory and efficient manner and at the same time treat large volumes of water and be user-friendly.

An aspect of currently used disinfection devices is, for example, that the desired degree of cleanliness is not always achieved. This may be the result of, for instance, the spray means not producing a spray pattern which covers the disinfection chamber and thus does not reach the entire disinfection chamber to the desirable degree and all the objects that are to be disinfected. In addition, dirt and particles may stick to the disinfection device, which means that the quality of cleaning is affected.

Moreover, existing disinfection devices may have the drawback that dirt and particles getting stuck during the disinfection process, such as residues from surgery, may be time consuming or difficult to remove.

It is thus desirable to provide an improved disinfection device.

JP 2005 328886 discloses a dishwasher with a washing shower arm for jetting rinsing liquid. The rinsing shower arm is provided with a shower arm main body part where a flow path for making the rinsing liquid flow is formed inside, and a nozzle part attachable and detachable to/from the shower arm main body part. The body section is equipped with a cap section which closes the edge of the shower arm. A breakthrough is located on the upper side of the nozzle part.

### Summary of the Invention

The object of the present invention is to provide a disinfection device with a spray arm which eliminates at least some of the above problems.

According to the present invention this and other objects are achieved with a disinfection device as defined in claim 1. By providing the nozzle device in this way, a spray arm and nozzle device that are user optimized is achieved. The nozzle device can easily be removed by the user and thus the spray arm can easily be cleaned. Accordingly, due to the detachably arrangement of the nozzle device the spray arms are cleanable.

Furthermore, a more efficient production of the spray arm is achieved. It is now possible to use the same manufacturing tools for different size of spray arms, since the nozzle device is assembled with the spray arm after the spray arm is produced. Accordingly, for different size of the disinfection devices, the same manufacturing tools can be used.

According to an embodiment of the invention, the opening of said outlet nozzle is oriented with its greatest extent transversely to the plane of rotation so that said outlet nozzle produces a spray pattern with a directional component in the longitudinal direction of the spray arm and with its greatest extent transversely to the plane of rotation. By providing the opening of said outlet nozzle in this way, an improved spray pattern is achieved. The improved spray pattern could be a flat spray pattern, but other spray patterns are possible.

Further said spray pattern has an extent that passes through the plane of rotation. By providing the opening of said outlet nozzle in this way, a further improved spray pattern is achieved. For example, is it possible for the sprays to reach spaces in a chamber of a disinfection device that otherwise are difficult to reach, such as the corners.

According to an embodiment of the invention, said outlet nozzle has an essentially slotted extent and thus produces a flat spray pattern with its orientation transversely to the plane of rotation.

According to an embodiment of the invention, said nozzle device has at least two outlet nozzles which, seen in the longitudinal direction of the spray arm, are two nozzles diverging transversely to the plane of rotation. By providing the outlet nozzles in this way, an essentially symmetrical configuration of the spray arm with the outlet nozzles, is achieved. Accordingly, the spray arm is more user optimized. It is easier for the user to attach the nozzle device with the spray arm again, after the user for examples has removed it for cleaning.

According to an embodiment of the invention, said plane of rotation is horizontal, that is the axis of rotation of the spray arm is vertical. By providing the plane of rotation horizontally, it is possible to obtain a washer disinfector with an essentially simple configuration. Moreover, the space in the chamber can be utilized more efficient, since the wash cart, the wash insert or wash basket usually are placed horizontally in the chamber. Further, depending on the position and configuration of the outlets of the spray arm, it is also possible to easily achieve a self-rotation of the spray arm, for instance by discharging the fluid in rotary assisting direction.

According to an embodiment of the invention, said spray arm is a tubular sectional element and said nozzle device is a cover means which seals the peripheral end of the tubular sectional element and in which said outlet nozzle is arranged. By providing the spray arm in this way, it is possible to achieve a manufacturing of the spray arm that is easy and cost-effective.

According to an embodiment of the invention, said tubular sectional element at least in its end portion has a constant longitudinal section. By providing the tubular sectional element with a constant longitudinal section, an efficient manufacturing is achieved. Accordingly it is easy to adapt the product depending on the size of the washer disinfector and disinfection chamber.

According to an embodiment of the invention, said cover means overlaps the end portion of the tubular sectional element. By providing the cover means in this way, a spray arm that is user oriented is achieved. That is, when the user is going to assemble the tubular sectional element with the cover means, for instance after cleaning the spray arm, it is easy for the user to se how the parts are going to be fit together.

According to an embodiment of the invention, the opening side, facing the tubular sectional element, of the cover means has a peripheral edge groove, whose width and extent correspond to the shape of the terminal edge portion of the tubular sectional element, which terminal edge portion thus is insertable into said groove.

According to an embodiment of the invention, a pivotable locking means is arranged to engage said cover means by snap action. By providing a locking means in this way, a reliable and satisfactory securing of the cover means is achieved.

According to an embodiment of the invention, said locking means is a U-shaped yoke which is designed, when pivoting from its unlocked position to its locked position, to pass a locking projection against spring action. By providing the locking means in this way, a locking means that is easy for the user to utilize is achieved. Accordingly, the user can smoothly remove the cover means for cleaning.

According to an embodiment of the invention, a spring catch extends from said cover means over the tubular sectional element, and a shoulder is arranged on the tubular sectional element to be grasped by the spring catch.

According to an embodiment of the invention, said spring catch is designed to engage said shoulder by snap action.

According to an embodiment of the invention, a flange projecting from the outside of the tubular sectional element and along the tubular sectional element is provided, the flange being formed with a recess whose lateral edge forms said shoulder.

According to an embodiment of the invention, said spring catch is formed by a projection projecting from the opening edge of the cover means and having a slot for receiving a flange portion positioned next to the end of the tubular sectional element.

### Brief Description of the Drawings

A currently preferred embodiment of the present invention will now be described in more detail, with reference to the accompanying schematic drawings.

Fig. 1 is an overview showing a disinfection apparatus with a spray arm according to an embodiment of the invention.

Fig. 2 is a schematic perspective view showing a spray arm provided with nozzles devices according to an embodiment of the invention.

Fig. 3 is an exploded schematic perspective view showing a spray arm with a nozzle device according to fig. 2.

Fig. 4 is a cross-sectional view showing a spray arm according to fig. 2.

Fig. 5 is a schematic perspective view showing a spray arm with a nozzle device according to an alternative embodiment of the invention.

### Detailed Description of a Preferred Embodiment

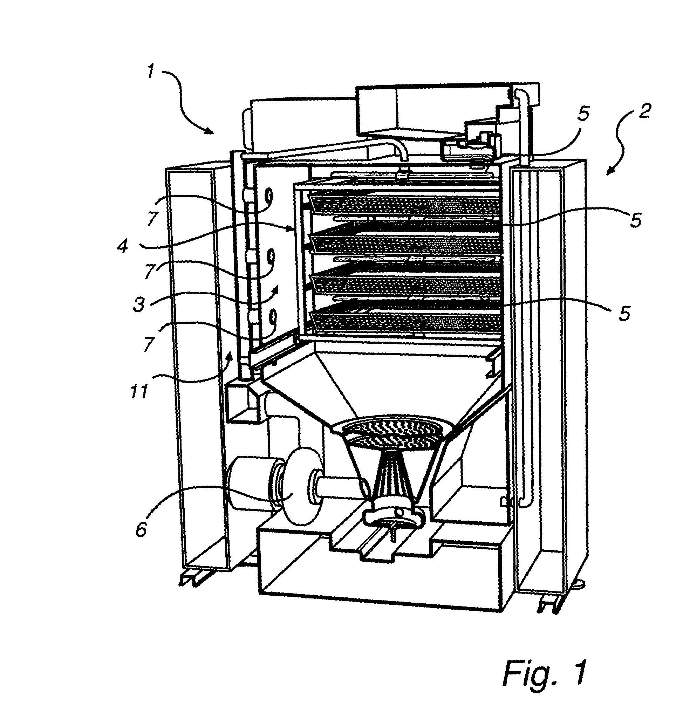

With initial reference to fig. 1, there is shown a perspective view of a washer disinfector 1 which has a housing 2 in which a disinfection chamber 3 is arranged. Examples of objects to be washed, disinfected and dried in a disinfection program are for instance surgical instruments, endoscopes, instruments for minimum invasive surgery, utensils, anaesthetic and respiratory equipment, as well as bottles, glass jars and hollowware. The chamber 3 is adapted to receive objects to be disinfected which objects may be arranged on a wash cart 4 or on an insert.

The wash cart 4 in the form of a basket rack with multiple levels is guided in the chamber 3 and may be liquid/fluid connected in an active position. In this case the chamber 3 exhibits three connecting parts 7 for supplying liquid or fluid via corresponding connecting parts at the wash cart 4. At least one spray arm 5 are rotatably and essentially horizontally arranged in the chamber 3, and in this embodiment five spray arms 5 are arranged at the upper and lower part of the chamber 3, and between the baskets at the wash cart 4. The wash cart 4 may be of different designs and functions, such as provided with injector heads or ramps adapted for the objects to be received.

A circulation pump 6 is provided for circulating the liquid being received in the chamber 3, by the circulation pipe 11 to the spray arms 5.

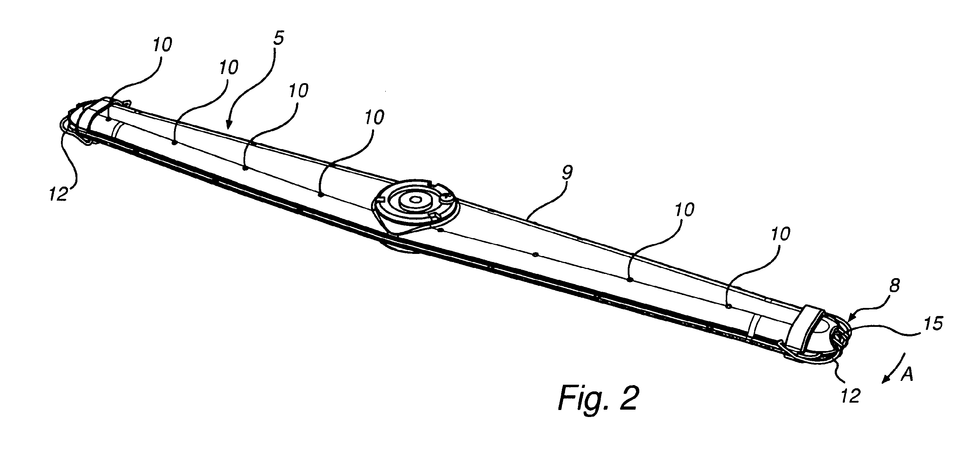

Fig. 2 shows a perspective view of an embodiment of a spray arm 5. In the figure the direction of rotation is as indicated by the arrow A. The spray arm 5 consist of a tubular sectional element 9 that is provided with a nozzles device 8 on each end of the spray arm 5. The nozzle device 8 is detachable arranged on the tubular sectional element 9.

In this preferred embodiment, each nozzle device 8 has two outlet nozzles 15 provided, seen in the rotary direction of the spray arm A, at the peripheral end portion of the spray arm 5.

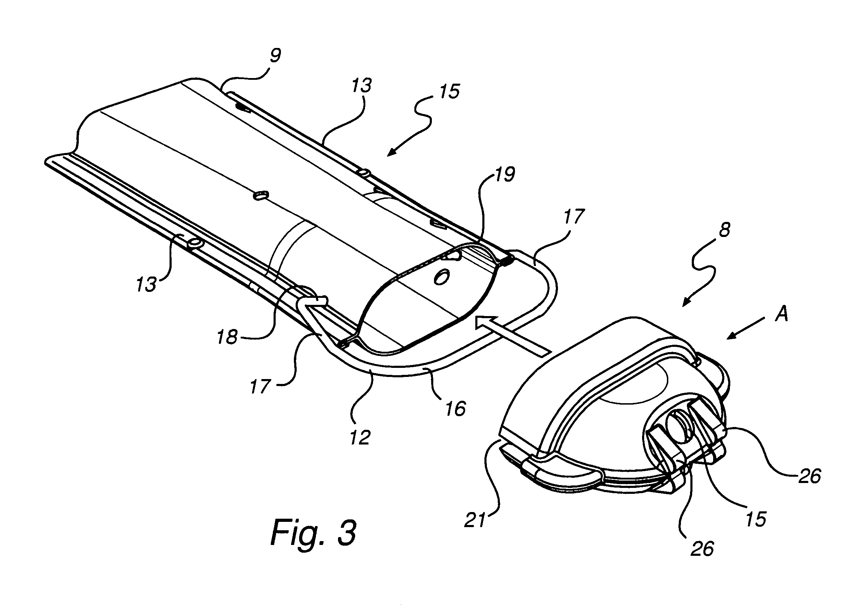

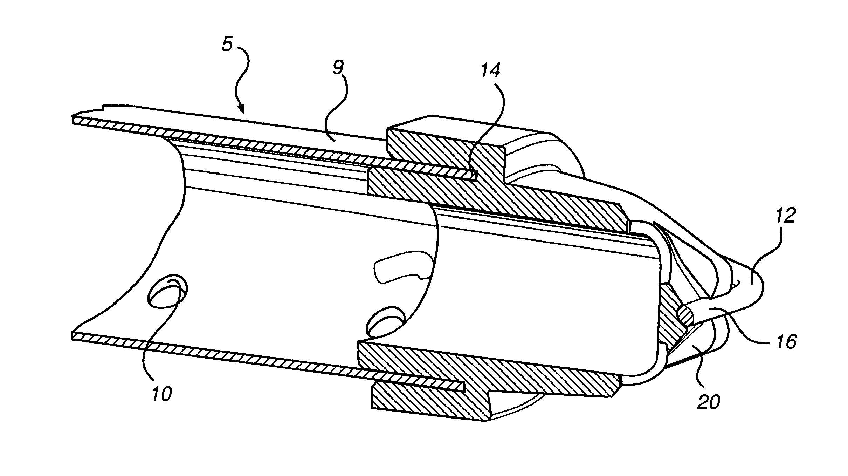

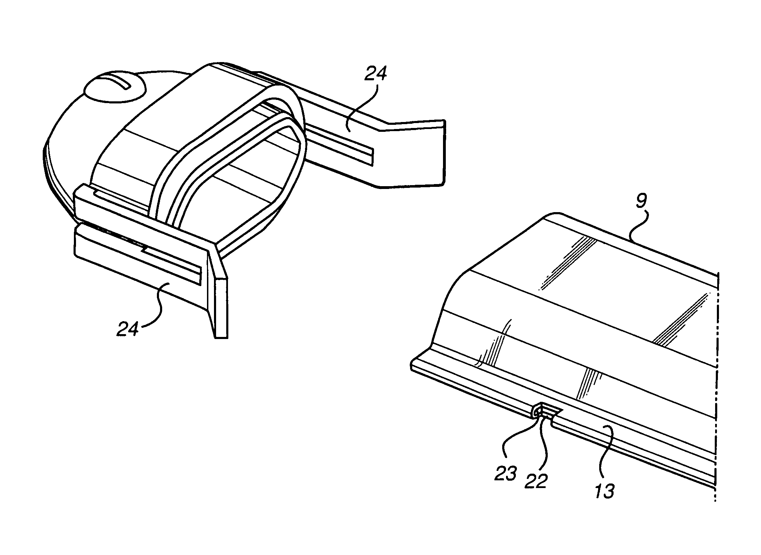

Further, a pivotable locking means is arrange on each end of the tubular sectional element 9 and in this preferred embodiment it is a U-shaped yoke 12. The yoke 12 interact with the nozzle device 8 such that the user easily can move the yoke 12 from its locked position to its unlocked position to detach the nozzle device 8 from the element 9. In fig. 2 the nozzle device 8 is in its locked position. The locking means will be described in more detail in fig. 3.

On the tubular sectional element 9 there are provided a couple of outlets 10 that are located on top of, on the side of, and underneath of the element 9. The outlets 10 distributing the liquid in the chamber after that the liquid has been circulated by the circulation pipe 11 through the connecting parts 7 to the spray arms 5. The outlets 10 are preferably arranged and directed such that at least one outlet or nozzle is assisting the rotation of the spray arm 5.

Further, the tubular sectional element 9 has a constant longitudinal section in both of its end portions. This is advantageously since the same manufacturing tools can be used for different size of the spray arms 5. Accordingly the same manufacturing tools can be used for different size and types of washer disinfectors.

Fig. 3 shows a part of the spray arm 5 according to fig. 2. In the figure the direction of rotation is as indicated by the arrow A. The nozzle device 8, that is a cover means, is in a disconnected position from the tubular sectional element 9.

The tubular sectional element 9 has a side edge portion 13, constituting a flange, extending in the longitudinal direction on each side of their both sides.

The yoke 12 consist of two legs 17 and an intermediate section 16 there between, that connecting the legs. The legs 17 has end portions 18 which are angled against each other and are inserted into a hole on each side of the tubular sectional element 9, and thereby constituting a pivoting hinge.

On each side of the outlet nozzles 15, a snap projection 26 is provided, which interact with the yoke 12 by snap action.

For disconnect the cover means from the tubular sectional element 9, for instance for cleaning the cover means, the user has moved the yoke 12 against spring action.

Accordingly, the user has moved the intermediate section 16 of the yoke 12 in the transverse direction and thereafter moved the cover means away from the element 9.

For connect the cover means with the tubular sectional element 9, the user will move the cover means over the terminal edge portions 19 of the tubular sectional element 9, when the yoke is turned in the transversally direction of the element 9.

Fig. 4 shows a cross-sectional view of the spray arm 5 when the locking means is in its locked position, accordingly the cover means is connected with the element 9.

The intermediate section 16 of the yoke 12 is steadily positioned in the snap projections 26. The terminal edge portions 19 of the tubular sectional element 9 are inserted in the edge groove 14 of the cover means.

The flange 13 protrudes through a gap 21 (shown in fig. 3) positioned in the side of the nozzle device 8. (not shown in the figure)

Fig. 5 shows perspective view of an alternative embodiment of the locking means. The locking means constitutes of a spring catch 24 that is provided on the cover means, and a recess 22 arranged on the flange 13 of the tubular sectional element 9. The lateral edge of the recess 22 forms a shoulder 23 and the spring catch 24 will engage the shoulder 23 for locking the cover means with the element 9.

It should be noted that different modifications of the embodiments of the invention described above are feasible within the scope of invention, as it is defined by the following claims.

For example it is possible to provide the opening of said outlet nozzle such as other spray patterns are achieved, for instance a round opening that achieves at least a partly circular spray pattern.

Furthermore, in the preferred embodiment the U-shaped yoke 12 is arranged transversal the longitudinal direction of the spray arm, i.e. in the direction of rotation. But it is also possible to arrange the yoke 12 in other directions, such as transversally the direction of rotation.

Rotations indicators is also possible to arrange in the washer device, for checking that the spray arm is rotating.

Furthermore, in the preferred embodiment the axis of rotation of the spray arm is vertical, but it is also possible to arrange the axis of rotation of the spray arm horizontally, i.e. the direction of rotation of the spray arm is transversally the direction of rotation in the preferred embodiment (shown in fig 3 by the arrow A).

## Claims

**1.** A disinfection device with a spray arm (5) and a shaft in a disinfection space, the spray arm being rotatable about the shaft and provided with outlet nozzles (15) for spraying the object that is to be disinfected, wherein said spray arm (5) has in its end portion a detachably arranged nozzle device (8), said nozzle device (8) having at least one outlet nozzle (15), wherein said spray arm (5) is a tubular sectional element (9) and said nozzle device (8) is a cover means which seals the peripheral end of the tubular sectional element and in which said outlet nozzle (15) is arranged;
said outlet nozzle (15) is arranged, seen in the rotary direction of the spray arm, at a peripheral end portion of the spray arm (5); and
the opening of said outlet nozzle (15) is oriented with its greatest extent transversely to the plane of rotation of the spray arm so that said outlet nozzle (15) produces a spray pattern with a directional component in the longitudinal direction of the spray arm (5) and with its greatest extent transversely to the plane of rotation of the spray arm.

**2.** A disinfection device as claimed in claim 1, wherein said spray pattern has an extent that passes through the plane of rotation.

**3.** A disinfection device as claimed in claim 1 or 2, wherein said outset nozzle (15) has a slotted extent and thus produces a flat spray pattern with its orientation transversely to the plane of rotation of the spray arm.

**4.** A disinfection device as claimed in any one of the preceding claims, wherein said nozzle device (8) has at least two outlet nozzles (15) which, seen in the longitudinal direction of the spray arm, are two nozzles diverging transversely to the plane of rotation.

**5.** A disinfection device as claimed in any one of the preceding claims, wherein said plane of rotation is horizontal, that is the axis of rotation of the spray arm (5) is vertical.

**7.** A disinfection device as claimed in any one of the preceding claims, wherein said tubular sectional element (9) at least in its end portion has a constant longitudinal section.

**8.** A disinfection device as claimed in any one of the preceding claims, wherein said cover means overlaps the end portion of the tubular sectional element.

**9.** A disinfection device as claimed in any one of the preceding claims, wherein the opening side, facing the tubular sectional element (9), of the cover means has a peripheral edge groove (14), whose width and extent correspond to the shape of the terminal edge portion (19) of the tubular sectional element (9), which terminal edge portion (19) thus is insertable into said groove (14).

**10.** A disinfection device as claimed in any one of the preceding claims, wherein a pivotable locking means is arranged to engage said cover means by snap action.

**11.** A disinfection device as claimed in claim 10, wherein said locking means is a U-shaped yoke (12) which is designed, when pivoting from its unlocked position to its locked position, to pass a locking projection against spring action.

**12.** A disinfection device as claimed in any one of claims 1 to 10, wherein a spring catch (24) extends from said cover means over the tubular sectional element (9), and a shoulder (23) is arranged on the tubular sectional element (9) to be grasped by the spring catch (24).

**13.** A disinfection device as claimed in claim 12, wherein said spring catch (24) is designed to engage said shoulder (23) by snap action.

**14.** A disinfection device as claimed in claim 12 or 13, wherein a flange (13) projecting from the outside of the tubular sectional element (9) and along the tubular sectional element is provided, the flange (13) being formed with a recess (22) whose lateral edge forms said shoulder (23).

**15.** A disinfection device as claimed in any one of claims 12-14, wherein said spring catch (24) is formed by a projection projecting from the opening edge of the cover means and having a slot for receiving a flange portion positioned next to the end of the tubular sectional element (9).

## Patentansprüche

**1.** Desinfektionsvorrichtung mit einem Sprüharm (5) und einer Welle in einem Desinfektionsraum, wobei der Sprüharm um die Welle herum drehbar ist und mit Auslassdüsen (15) zum Besprühen des zu desinfizierenden Gegenstandes versehen ist, wobei der Sprüharm (5) in seinem Endabschnitt eine abnehmbar angeordnete Düsenvorrichtung (8) aufweist, wobei die Düsenvorrichtung (8) mindestens eine Auslassdüse (15) aufweist, wobei der Sprüharm (5) ein Element mit röhrenförmigem Profil (9) ist und die Düsenvorrichtung (8) ein Abdeckmittel ist, welches das periphere Ende des Elements mit röhrenförmigem Profil abdichtet und in dem die Auslassdüse (15) angeordnet ist;
wobei die Auslassdüse (15), in der Drehrichtung des Sprüharms betrachtet, an einem peripheren Endabschnitt des Sprüharms (5) angeordnet ist; und
die Öffnung die Auslassdüse (15) mit ihrer größten Erstreckung quer zur Rotationsebene des Sprüharms ausgerichtet ist, so dass die Auslassdüse (15) ein Sprühmuster mit einer Richtungskomponente in der Längsrichtung des Sprüharms (5) und mit seiner größten Erstreckung quer zur Rotationsebene des Sprüharms erzeugt.

**2.** Desinfektionsvorrichtung nach Anspruch 1, wobei das Sprühmuster eine Erstreckung aufweist, die durch die Rotationsebene hindurch verläuft.

**3.** Desinfektionsvorrichtung nach Anspruch 1 oder 2, wobei die Auslassdüse (15) eine schlitzartige Erstreckung aufweist und **dadurch** ein flaches Sprühmuster mit seiner Ausrichtung quer zur Rotationsebene des Sprüharms erzeugt.

**4.** Desinfektionsvorrichtung nach einem der vorangehenden Ansprüche, wobei die Düsenvorrichtung (8) mindestens zwei Auslassdüsen (15) aufweist, die, in der Längsrichtung des Sprüharms betrachtet, zwei Düsen sind, die quer zur Rotationsebene divergieren.

**5.** Desinfektionsvorrichtung nach einem der vorangehenden Ansprüche, wobei die Rotationsebene horizontal verläuft, das heißt, die Rotationsachse des Sprüharms (5) verläuft vertikal.

**7.** Desinfektionsvorrichtung nach einem der vorangehenden Ansprüche, wobei das Element mit röhrenförmigem Profil (9) mindestens in seinem Endabschnitt ein konstantes Längsprofil aufweist.

**8.** Desinfektionsvorrichtung nach einem der vorangehenden Ansprüche, wobei das Abdeckmittel den Endabschnitt des Elements mit röhrenförmigem Profil überlappt.

**9.** Desinfektionsvorrichtung nach einem der vorangehenden Ansprüche, wobei die dem Element mit röhrenförmigem Profil (9) zugewandte Öffnungsseite des Abdeckmittels eine periphere Randnut (14) aufweist, deren Breite und Erstreckung der Gestalt des Endrandabschnitts (19) des Elements mit röhrenförmigem Profil (9) entspricht, wodurch dieser Endrandabschnitt (19) in die Nut (14) eingesetzt werden kann.

**10.** Desinfektionsvorrichtung nach einem der vorangehenden Ansprüche, wobei ein schwenkbares Verriegelungsmittel so angeordnet ist, dass es auf das Abdeckmittel aufgeschnappt werden kann.

**11.** Desinfektionsvorrichtung nach Anspruch 10, wobei das Verriegelungsmittel ein U-förmiges Joch (12) ist, das so gestaltet ist, dass es, wenn es aus seiner unverriegelten Position in seine verriegelte Position schwenkt, einen Verriegelungsvorsprung entgegen einer Federwirkung passiert.

**12.** Desinfektionsvorrichtung nach einem der Ansprüche 1 bis 10, wobei sich ein Schnappverschluss (24) von dem Abdeckmittel über das Element mit röhrenförmigem Profil (9) erstreckt und eine Schulter (23) an dem Element mit röhrenförmigem Profil (9) angeordnet ist, die durch den Schnappverschluss (24) erfasst wird.

**13.** Desinfektionsvorrichtung nach Anspruch 12, wobei der Schnappverschluss (24) dafür ausgelegt ist, auf die Schulter (23) aufgeschnappt zu werden.

**14.** Desinfektionsvorrichtung nach Anspruch 12 oder 13, wobei ein Flansch (13), der von der Außenseite des Elements mit röhrenförmigem Profil (9) und entlang dem Element mit röhrenförmigem Profil hervorsteht, angeordnet ist, wobei der Flansch (13) mit einer Aussparung (22) versehen ist, deren seitlicher Rand die Schulter (23) bildet.

**15.** Desinfektionsvorrichtung nach einem der Ansprüche 12-14, wobei der Schnappverschluss (24) durch einen Vorsprung gebildet wird, der von der Öffnungskante des Abdeckmittels hervorsteht und einen Schlitz aufweist, um einen Flanschabschnitt aufzunehmen, der sich neben dem Ende des Elements mit röhrenförmigem Profil (9) befindet.

## Revendications

**1.** Dispositif de désinfection comportant un bras d'arrosage (5) et un arbre dans un espace de désinfection, le bras d'arrosage étant rotatif autour de l'arbre pourvu de buses de sortie (15) pour arroser l'objet qui doit être désinfecté, dans lequel ledit bras d'arrosage (5) possède dans sa portion d'extrémité un dispositif de buse (8) disposé de manière amovible, ledit dispositif de buse (8) possédant au moins une buse de sortie (15), dans lequel ledit bras d'arrosage (5) est un élément sectionnel tubulaire (9) et ledit dispositif de buse (8) est un moyen de couvercle qui scelle l'extrémité périphérique de l'élément sectionnel tubulaire et dans lequel ladite buse de sortie (15) est agencée;
ladite buse de sortie (15) est agencée, vue dans la direction rotative du bras d'arrosage, à une portion d'extrémité périphérique du bras d'arrosage (5); et l'ouverture de ladite buse de sortie (15) est orientée avec son extension la plus grande transversalement au plan de rotation du bras d'arrosage, de telle sorte que ladite buse de sortie (15) produise un modèle d'arrosage avec un composant directionnel dans la direction longitudinale du bras d'arrosage (5) et avec sa plus grande extension transversalement au plan de rotation du bras de pulvérisation.

**2.** Dispositif de désinfection selon la revendication 1, dans lequel ledit modèle d'arrosage a une extension qui passe à travers le plan de rotation.

**3.** Dispositif de désinfection selon la revendication 1 ou 2, dans lequel ladite buse de sortie (15) a une extension rainurée et produit ainsi un modèle d'arrosage plat avec son orientation transversalement au plan de rotation du bras d'arrosage.

**4.** Dispositif de désinfection selon une quelconque des revendications précédentes, dans lequel ledit dispositif de buse (8) possède au moins deux buses de sortie (15), lesquelles, vues dans la direction longitudinale du bras d'arrosage, sont deux buses divergeant transversalement au plan de rotation.

**5.** Dispositif de désinfection selon une quelconque des revendications précédentes, dans lequel ledit plan de rotation est horizontal, c'est à dire que l'axe de rotation du bras d'arrosage (5) est vertical.

**7.** Dispositif de désinfection selon une quelconque des revendications précédentes, dans lequel ledit élément sectionnel tubulaire (9) possède une section longitudinale constante au moins dans sa portion d'extrémité.

**8.** Dispositif de désinfection selon une quelconque des revendications précédentes, dans lequel ledit moyen de couvercle se superpose à la portion d'extrémité de l'élément sectionnel tubulaire.

**9.** Dispositif de désinfection selon une quelconque des revendications précédentes, dans lequel le côté d'ouverture, faisant face à l'élément sectionnel tubulaire (9), du moyen de couvercle possède une gorge de bord périphérique (14), dont la largeur et l'extension correspondent à la forme de la portion de bord terminal (19) de l'élément sectionnel tubulaire (9), laquelle portion de bord terminal (19) peut ainsi être insérée dans ladite gorge (14).

**10.** Dispositif de désinfection selon une quelconque des revendications précédentes, dans lequel un moyen de verrouillage pivotant est disposé afin de venir en prise avec ledit moyen de couvercle par action d'encliquetage.

**11.** Dispositif de désinfection selon la revendication 10, dans lequel ledit moyen de verrouillage est une fourche en forme de U (12) qui est conçue, lorsqu'elle pivote de sa position déverrouillé à sa position verrouillée, afin de passer une protubérance de verrouillage en sens contraire à l'action du ressort.

**12.** Dispositif de désinfection selon une quelconque des revendications 1 à 10, dans lequel une fermeture à ressort (24) s'étend depuis ledit moyen de couvercle par dessus l'élément sectionnel tubulaire (9), et un épaulement (23) est disposé sur l'élément sectionnel tubulaire (9) afin d'être saisi par la fermeture à ressort (24).

**13.** Dispositif de désinfection selon la revendication 12, dans lequel ladite fermeture à ressort (24) est conçue afin de venir en prise avec ledit épaulement (23) par action d'encliquetage.

**14.** Dispositif de désinfection selon la revendication 12 ou 13, dans lequel une bride (13) se projetant depuis l'extérieur de l'élément sectionnel tubulaire (9) et le long de l'élément sectionnel tubulaire est prévue, la bride (13) étant formée avec une cavité (22) dont le bord latéral forme ledit épaulement (23).

**15.** Dispositif de désinfection selon une quelconque des revendications 12 à 14, dans lequel ladite fermeture à ressort (24) est formée par une protubérance se projetant depuis le bord d'ouverture du moyen de couvercle et possédant une rainure pour recevoir une portion de bride positionnée près de l'extrémité de l'élément sectionnel tubulaire (9).
